# EUROPÄISCHE PATENTSCHRIFT

(11) **EP 3 927 712 B1**
(45) Veröffentlichungstag und Bekanntmachung des Hinweises auf die Patenterteilung: **03.08.2022**
(21) Anmeldenummer: 20711034.7
(22) Anmeldetag: 03.02.2020
(51) Int. Cl.: C07F 7/18, C07C 39/23

(54) **VERFAHREN ZUR HERSTELLUNG VON CANNABIDIOL**
METHOD FOR PREPARING CANNABIDIOL
PROCÉDÉ DE PRODUCTION DE CANNABIDIOL

(30) Priorität: 22.02.2019 DE 102019104563
(43) Veröffentlichungstag der Anmeldung: 29.12.2021
(73) Patentinhaber: Chiracon GmbH, 14943 Luckenwalde (DE)
(72) Erfinder: ZUHSE, Ralf, 12279 Berlin (DE); OSTROVSKYI, Dmytro, 14167 Berlin (DE)
(74) Vertreter: Castell, Klaus
(86) Internationale Anmeldenummer: PCT/DE2020/000015
(87) Internationale Veröffentlichungsnummer: WO 2020/169135

(56) Entgegenhaltungen:
- US-A1- 2017 008 868

## Beschreibung

Die Erfindung betrifft ein Verfahren zur Herstellung von Cannabidiol und eine Zwischenstufe zur Herstellung von Cannabidiol, wobei zwei Zwischenstufen erhalten werden, nämlich ein silyliertes Olivetol und ein silyliertes und bromiertes Olivetol die stabil, lager- und verkehrsfähig sind.

Zur Herstellung von Medikamenten, die auf einem chiralen Wirkstoff basieren, ist es wichtig, bei der Synthese diese nach Möglichkeit enantiomerenrein herzustellen. Da unterschiedliche Enantiomere eines chiralen Wirkstoffs sehr erheblich voneinander abweichende pharmakologische Wirkungen entfalten können, kann bereits eine geringe Kontamination des chiralen Wirkstoffs mit dem unerwünschten Enantiomer beim Patienten zu sehr erheblichen, auch bei strenger Indikation nicht mehr akzeptablen Nebenwirkungen führen. Seit einer pharmakologischen Katastrophe in den 1960er Jahren, als ein Wirkstoff schwangeren Frauen verabreicht wurde, der selbst unter dem Thalidomid bekannt wurde, ist es bekannt, das verschiedene Enationmere der ansonsten gleichen Substanz sehr unterschiedliche Wirkungen haben können. Die Erforschung dieser Katastrophe ergab, dass die erwünschte sedierende Wirkung dem (+)-(R)-Enantiomeren zukommt, die fruchtschädigende Wirkung jedoch dem (-)-(S)-Enantiomeren zugeschrieben wird. Die Besonderheit dieses Wirkstoffes ist es, dass beide Enantiomere sich im Körper innerhalb von ca. acht Stunden ineinander umwandeln, indem sie racemisieren. Andere chirale Wirkstoffe, die im Körper ohne vorherige Racemisierung metabolisiert werden, können auch sehr unterschiedliche Wirkprofile zeigen.

Zur Herstellung chiraler Wirkstoffe verfolgt man daher unterschiedliche Synthesestrategien, um enantiomerenreine Wirkstoffe zu erhalten. Dabei werden im Wesentlichen drei Wege verfolgt.

Ein erster Weg geht von einer enantiomerenreinen chiralen Substanz aus, von der aus das erwünschte Molekül synthetisiert wird. Dieses Syntheseroute erfordert Synthesewege, die eine Racemisierung eines in der Synthesestrategie erwünschten Zwischenproduktes unterdrücken oder zumindest nicht fördern. Bei der Synthese wird das eine chirale Zentrum oder auch die chiralen Zentren nicht mehr verändert oder durch einen enantioselektiven Schritt umgesetzt. Die Kontrolle solcher Synthesestrategien ist häufig nicht leicht und notwendige Zwischenschritte können das chirale Zentrum zur Racemisierung veranlassen.

Ein zweiter Weg zur enantiomenrereinen Synthese führt über die Herstellung des Racemats, wobei das Racemat durch Verwendung von chiralen Hilfsmitteln in seine Enantiomere aufgetrennt wird. Chirale Hilfsmittel können Chromatografische Methoden umfassen, bei denen die stationäre Phasen der Chromatografiesäule selbst enantiomerenrein sind und beispielsweise aus biologisch gewonnenem Material bestehen. Verfahren, welche diese Synthesestrategie verfolgen, nehmen von vornherein eine Ausbeute von Weniger als 50% in Kauf, weil mindestens 50% als Enantiomer einer Substanz verloren ist. Sofern sich das nicht erwünschte Enantiomer durch den Einsatz von extremeren Bedingungen als bei der Synthese racemisieren lässt, so könnte das reine Enantiomer durch stetige Abscheidung des erwuünschten Enantiomers aus einem stets neu racemisierten Abfallprodukt der Enantiomerentrennung erhalten. Ein solcher Weg ist in der Regel wirtschaftlich sehr aufwändig, weil die Abscheidung und Racemisierung eines im Kreislauf befindlichen Produktes mit stets geringeren Ausbeuten erhalten lässt. Selbst wenn man das Racemat im Kreislauf führt, so ist man auf sehr erheblichen Einsatz von chiralen Hilfsmitteln angewiesen, die in der Regel wirtschaftlich erhebliche wirtschaftliche Mittel erfordern.

Ein Dritter Weg führt schließlich über den Einsatz von Enzymen biologischer Herkunft, welche aus einem prochiralen Zwischenprodukt durch eine enantioselektive Umsetzung das erwünschte enantiomerenreine Produkt erzeugen. Sofern es gelingt, das ausgewählte Enzym an einer Stelle einzusetzen, an der aus einem prochiralen Zentrum des Zwischenproduktes mit hoher Selektivität das neue, entiomere Produkt entsteht, ist dieser Weg häufig ein bevorzugter Weg zur Synthese. Tatsächlich aber ist nicht jede Synthesestrategie fuür den Einsatz von bekannten Enzymen geeignet. Eine weitere, nicht selten auftretende Komplikation ist, dass das prochirale Zentrum für den Einsatz des Enzyms reaktiv ist und von daher auch spontan, also ohne enzymatische Katalyse reagieren kann und dabei racemisiert. Das racemische Produkt wird so an der enzymatischen Katalyse vorbei in das Endprodukt mitgerissen, wo es den erwünschten enantiomeren Exzess (ee) schmälert.

Ein neuerer pharmakologischer Wirkstoff, der chiral ist, ist Cannabidiol (CBD). Cannabidiol ist ein kaum psychoaktives Cannabinoid aus dem weiblichen Hanf. Medizinisch wirkt es entkrampfend, entzündungshemmend, angstlösend und gegen Übelkeit. Weitere pharmakologische Effekte wie z. B. eine antipsychotische Wirkung, werden zur Zeit dieser Anmeldung erforscht. Da vermutet wird, dass CBD eine Vielfalt von erwünschten Wirkungen im menschlichen Körper entfaltet, wenn es angemessen verabreicht wird, ist man bestrebt, CBD nach Möglichkeit synthetisch herzustellen, um es verunreinigungsfrei zu erhalten.

In der internationalen Patentanmeldung WO 2017011210 A1 und in der Anmeldung US 2017/008868 A1 wird ein Verfahren zur Herstellung von Dibromoolivetol gelehrt. Dibromoolivetol In dem dort gelehrten Verfahren wird jedoch eine Bromierung mit elementarem Brom bei tiefer Temperatur durchgeführt. Sowohl der Umgang mit lementarem Brom als auch die Durchführung der Reaktion bei tiefer Temperatur macht das Verfahren nicht nur unsicher in der Handhabung, sondern auch noch recht teuer bei der Überführung des Herstellungsverfahrens in den Technikums-Maßstab oder in noch größerem Maßstab.

Aufgabe der Erfindung ist es daher, ein Verfahren zur Herstellung von Cannabidiol zur Verfügung zu stellen, welches handhabungssicher und leichter skalierbar ist als es im Stand der Technik möglich ist. Die erfindungsgemäße Aufgabe gelöst durch das Verfahren nach Anspruch 1. Weitere vorteilhafte Ausgestaltungen sind in den Unteransprüchen zu Anspruch 1 angegeben. Des Weiteren wird eine stabile Zwischenstufe nach Anspruch 7 und Anspruch 8 beansprucht.

Das erfindungsgemäße Verfahren zeichnet sich aus durch eine Silylierung der Hydroxygruppe des als Edukt eingesetzten Olivetols und Maskierung von dessen Parastellung im aromatischen System. Dadurch verbleibt nur eine einzige Kopplungsmöglichkeit für eine weitere Umsetzung mit Menthadienol, was die Synthese handhabungssicher und spezifisch macht und, dass sich die Umsetzung bei Raumtemperatur durchführen lässt. Das ermöglicht eine Skalierung der Synthese.

Die erfindungsgemäße Synthese wird im Detail anhand der folgenden Versuchsbeschreibung erläutert.

### 1. Synthese von Di-OTBS-Olivetol

Der erste Syntheseschritt zur Herstellung von Cannabidiol erfolgt durch Silylieren von Olivetol mit TBSCI (tert-Butyldimethylsilylchlorid).

Dabei kann das Olivetol an der Pentlykette an den Stellen von R substituiert sein mit einer oder mehreren Substituenten bestehend aus Wasserstoff (H), Deuterium (D) oder Tritium (T), Alkoxy, wie Methoxy (-OCH₃), Ethoxy (-OC₂H₅), Nitro (-NO₂), Fluor (-F), Chlor (-CI), Brom (-Br), Alkyl von Methyl, Ethyl, Propyl bis Butyl. Der erste Syntheseschritt wird anhand des unsubstituierten Olivetol (R = H) beschrieben.

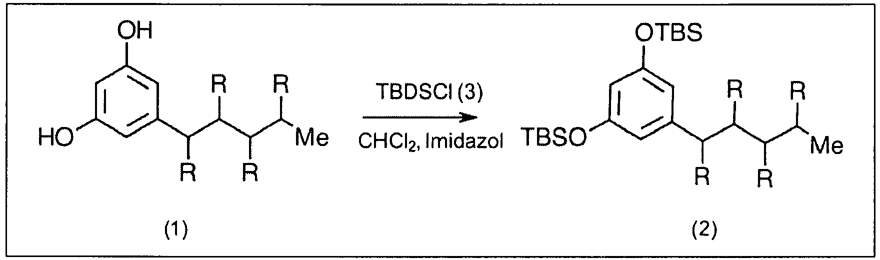

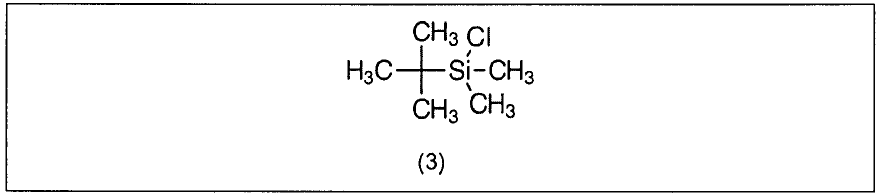

4,0108 g Olivetol (5-Pentylresorcin) (1) und 3,6330 g Imidazol (2,4 Äquivalente) (1,3-Diaza-2,4-cyclopentadien) werden in 48 ml Dichlormethan (CH₂Cl₂) gelöst, und 7,7864 g TBSCI (*tert*-Butyldimethylsilylchlorid) (3) werden zu der erhaltenen Lösung portionsweise zugegeben.

Die entstehende Suspension wird bei Raumtemperatur für 16 Stunden gerührt. Anschließend werden 30 ml VE-Wasser (VE-Wasser, vollentsalztes Wasser) zugegeben. Es bildet sich eine Emulsion, dessen Feststoff wird in der wässrigen Phase gelöst wird. Die Emulsion wird für mindestens 30 Minuten bei Raumtemperatur gerührt. Die Phasen sind nach angegebener Zeit getrennt. Die wässrige Phase wird zweimal mit 40 ml Dichlormethan (CH₂Cl₂) extrahiert. Die vereinten organischen Phasen werden mit 40 ml VE-Wasser gewaschen und mit Natriumsulfat (NaSO₄) getrocknet. Die resultierende Suspension wird über eine Filterfritte abgesaugt, und der Feststoff wird mit 15 ml Dichlormethan (CH₂Cl₂) gewaschen.

Das Filtrat wird sodann bei einer Badtemperatur von 40°C bis Enddruck von 12 mbar eingeengt. 9,2158 g Endprodukt kann isoliert werden (100% Ausbeute = 9,0964 g).

Das so erhaltene 3,5-Di-(Di-O-*tert*-Butyldimethylsilyl) -5-Pentylresorcin (2) ist stabil, lager- und transportfähig und eignet sich als verkehrsfähiges Edukt zur Herstellung von gegebenenfalls substitutierten Cannabidiolen.

Das Produkt, Di-O-*tert*-Butyldimethylsilyl-5-Pentylresorcin (2), wird ohne weitere Aufreinigung weiter umgesetzt.

### 2. Synthese von Di-OTBS-dibromolivetol

Der zweite Syntheseschritt erfolgt durch Bromieren des im vorhergehenden Schritt erhaltenen Di-O-*tert*-Butyldimethylsilyl-5-Pentylresorcins (2).

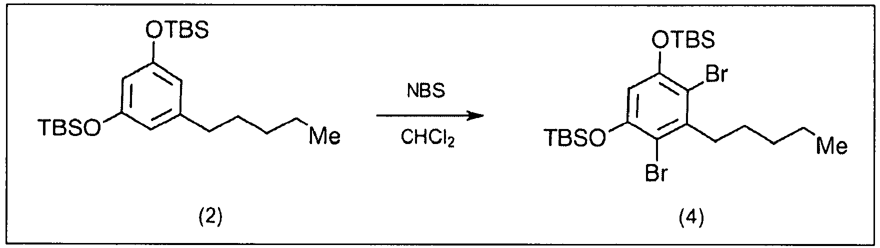

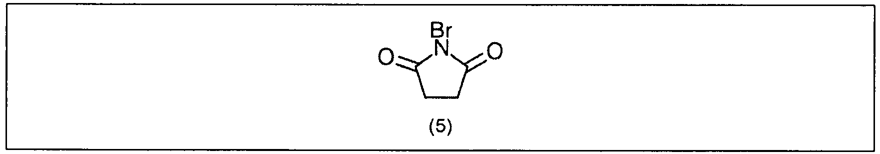

9,2158 g rohes Di-OTBS-Olivetol (Di-O-*tert*-Butyldimethylsilyl-5-Pentylresorcin) (2) werden in 90 ml Dichlormethan (CH₂Cl₂) gelöst. Die erhaltene Lösung wird bis auf 0°C gekühlt. 8,7126 g N-Bromosuccinimid (NBS, 2,2 Äquivalente) (5) werden unter Rühren portionsweise zugegeben. Sodann wird eine externe Kühlung entfernt und die entstehende Suspension wird bis Erreichen von Raumtemperatur erwärmt. Zugegebenes NBS löst sich langsam mit der Zeit. Nach 2 Stunden 5 Minuten entsteht eine klare Lösung. Zuletzt fällt ein feiner weißer Feststoff nach zusätzliche 15 Minuten Rührzeit aus. Eine Reaktionskontrolle mittels Dünnschichtchromatographie zeigt, dass fast 100% Umsatz stattfindet. Die Suspension wird für weiter 17 Stunden gerührt. Anschließend wird die Suspension über eine Filterfritte abgesaugt, und der Filterkuchen wird mit 10 ml Dichlormethan (CH₂Cl₂) gewaschen. Das erhaltene Filtrat wird sodann am Rotationsverdampfer bei einer Badtemperatur von 40°C eingeengt. Es entsteht ein Gemisch aus einem Öl und Feststoff. Das Gemisch wird mit 50 ml Cyclohexan (C₆H₁₂) versetzt und wird kurz bei Raumtemperatur gerührt. Die Reste von NBS werden abfiltriert und das Filtrat wird am Rotationsverdampfer bei einer Badtemperatur von 40°C bis Enddruck von 13 mbar eingeengt. Es wurden 12,2000g Endprodukt isoliert (Ausbeute = 96,8%).

1H-NMR-Spektrum zeigt nur das Auftreten von TBSCI-Spuren als einzige Verunreinigung.

Das so erhaltene Di-OTBS-dibromolivetol (4,6-Dibrom-di-O-*tert-*Butyldimethylsilyl-5-Pentylresorcin) (4) ist stabil, lager- und transportfähig und eignet sich als verkehrsfähiges Edukt zur Herstellung von gegebenenfalls substitutierten Cannabidiolen.

### 3. Synthese von Dibromolivetol

Im dritten Syntheseschritt wird das im vorhergehenden Schritt erhaltene Di-OTBS-dibromolivetol (4,6-Dibrom-di-O-*tert*-Butyldimethylsilyl-5-Pentylresorcin) (4) desilyliert.

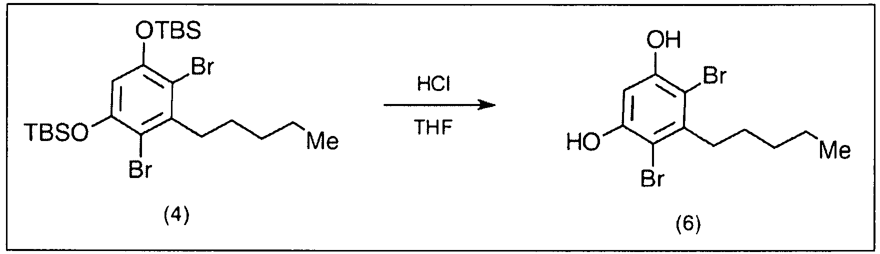

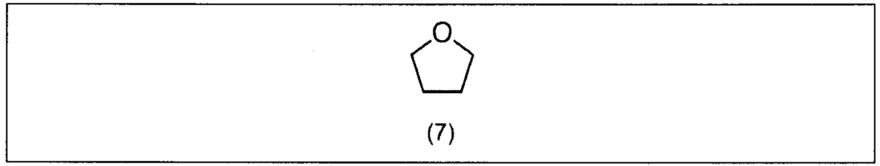

25,23 g Di-OTBS-dibromolivetol (4,6-Dibrom-di-O-*tert*-Butyldimethylsilyl-5-Pentylresorcin) (4) werden in 150 ml Tetrahydrofuran (C₄H₈O) gelöst und 11 ml VE-Wasser und 46 ml 25%-ige Salzsäure unter Rühren zugegeben. Es entsteht eine 2-phasige Emulsion. Die Emulsion wird bei Raumtemperatur gerührt, bis eine klare Lösung entsteht. Die Reaktionskontrolle mittels Dünnschichtchromatographie zeigt nach 71 Stunden 100% Umsatz. 77 ml 15%-ige Natronlauge werden über 20 Minuten verteilt tropfenweise zugegeben bis der pH -Wert von 2 bis 3 erreicht wird. Es entsteht eine 2-phasige Emulsion. Die Phasen werden getrennt und die wässrige Phase wird zweimal mit 150 ml tert-Butylmethylether extrahiert. Die vereinten organischen Phasen werden mit 100 ml Kochsalz-Lösung gewaschen und mit Natriumsulfat getrocknet. Die resultierende Suspension wird über eine Filterfritte abgesaugt, und der Filterkuchen wird zweimal mit 20 ml *tert-*Butylmethylether gewaschen. Das Filtrat wird am Rotationsverdampfer bei einem Badtemperatur von 40°C bis Enddruck von 18 mbar eingeengt. Das erhaltene Rohprodukt wird in 60 ml Cyclohexan gelöst. 1,2 ml VE-Wasser werden tropfenweise zu der Cyclohexan-Lösung unter Rühren zugegeben. Es bildet sich ein Niederschlag. Die Suspension wird gekühlt bis die Innentemperatur den Wert von 5 bis 10°C erreicht. Das gekühlte Gemisch wird für weiter 10 Minuten gerührt. Der Feststoff wird abgesaugt, zweimal mit 10 ml Cyclohexan gewaschen und bei Raumtemperatur an der Luft getrocknet. Es können 8,5352 g Endprodukt isoliert (Ausbeute = 57%) werden.

1H-NMR-Spektrum zeigt, dass keine Verunreinigungen vorliegen.

### 4. Synthese von Dibromocannabidiol

Im vierten Syntheseschritt wird das im vorhergehen Schritt erhaltene Dibromolivetol (4,6-Dibrom-5-Pentylresorcin) (6) durch Umsetzung mit trans-Menthadienol (4R-Isopropenyl-1-methylcyclohex-2-enol) (8) in Dibromocannabidiol (9) überführt.

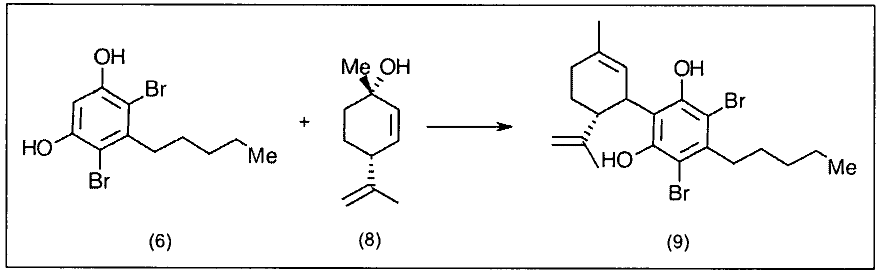

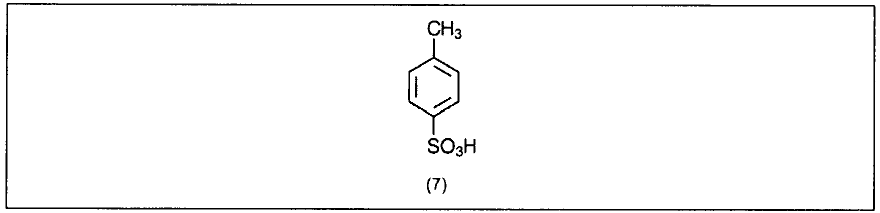

3,4028 g Dibromolivetol (4,6-Dibrom-5-Pentylresorcin) (6) werden in 51 ml Dichlormethan (CH₂Cl₂) gelöst. Die erhaltene Lösung wird am Rotationsverdampfer bei einer Badtemperatur von 40°C bis Enddruck von 15 mbar eingeengt. Das erhaltene Öl verfestigt sich schnell während der Abkühlung.

Der Feststoff wird mit 3,5323 g Magnesiumsulfat (MgSO₄) und 0,9614 g *para-*Toluolsulfonsäure-Monohydrat (pTSA, 0,5 Äquivalent) (7) gemischt. Sodann wird das Gemisch mit 25 ml Dichlormethan (CH₂Cl₂) versetzt. Es entsteht eine Suspension. Die Suspension wird einem Ethanol/Stickstoff-Bad bis zu einer Temperatur von -20°C bis -10°C abgekühlt.

2,1432 g trans-Menthadienol (4R-Isopropenyl-1-methylcyclohex-2-enol) (1,4 Äquivalente) (8) werden in 9 ml Dichlormethan (CH₂Cl₂) gelöst und die erhaltene Lösung wird zum Reaktionsgemisch tropfenweise über 12 Minuten verteilt zugegeben. Das Reaktionsgemisch wird 5 Stunden lang gerührt und anschließend in einem Tiefkühlschrank bei T = -25°C übernacht gelagert. Das Gemisch wird danach ohne Erwärmen mit 10 ml Natriumhydrogencarbonat-Lösung versetzt. Die entstehende Emulsion wird bis Raumtemperatur erwärmt und mit 20 ml VE-Wasser verdünnt. Die Phasen werden getrennt. Die wässrige Phase wird zweimal mit 50 ml Dichlormethan (CH₂Cl₂) extrahiert. Die vereinten organischen Phasen werden mit 30 ml Kochsalz-Lösung gewaschen und mit Natriumsulfat (Na₂SO₄) getrocknet. Die entstehende Suspension wird über eine Filterfritte abgesaugt. Der Filterkuchen wird dreimal mit 10 ml Dichlormethan (CH₂Cl₂) gewaschen und das Filtrat wird am Rotationsverdampfer bei einem Badtemperatur von 40°C bis Enddruck von 12 mbar eingeengt. Es werden 5,2315 g Rohprodukt erhalten. Das Rohprodukt wird mit 10 ml Cyclohexan (C₆H₁₂) versetzt. Es entsteht eine Lösung. Die Lösung wird bis auf eine Innentemperatur von 5 bis 10°C abgekühlt und 0,04 ml VE-Wasser werden unter Rühren zugegeben. Es bildet sich ein Niederschlag am Kolbenrand. Die entstehende Suspension wird für weitere 25 Minuten gerührt und der Feststoff wird abfiltriert und mit 3 ml Cyclohexan (C₆H₁₂) gewaschen. Das Filtrat wird am Rotationsverdampfer bei einem Badtemperatur von 40°C bis Enddruck von 12 mbar eingeengt. Es können 4,7400 g Zielprodukt isoliert werden.

1H-NMR-Spektrum zeigt ca. 85%ige Reinheit.

### 5. Synthese von Cannabidiol (CBD)

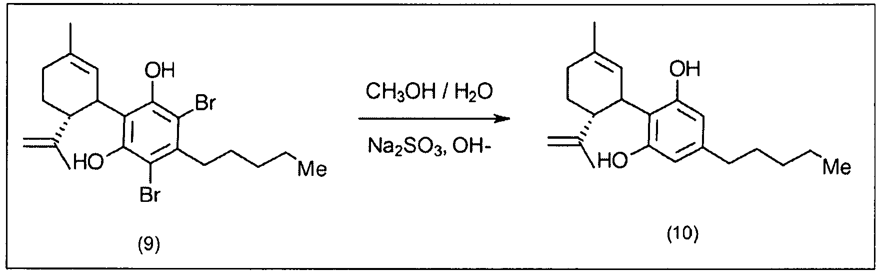

4,74 g Rohes Dibromcannabidiol (9) werden mit 47 ml Methanol (CH₃OH) versetzt. Es entsteht eine sehr dünne Suspension. 7,5912 g Natriumsulfit (Na₂SO₃) (6 Äquivalente) werden in 50 ml VE-Wasser gelöst und die Lösung wird zum Reaktionsgemisch zugegeben. Es bildet sich ein weißer Niederschlag. 3 ml 25%-ige Ammoniak-Lösung (ca. 4 Äquivalente) werden nach 30 Minuten unter Rühren dem Reaktionsgemisch zugegeben.

Die Reaktionskontrolle mittels Dünnschichtchromatographie (DC) zeigt nach 19 Stunden Rührzeit kein Endprodukt im Gemisch und Auftreten eines Monobrom-Derivats.

Zusätzliche 0,75 ml Ammoniak-Lösung (NH₃ / H₂O) werden zugegeben und die Suspension wird bis T = 60°C erhitzt. Nach 19 Stunden zeigt DC 100%ige Umsatz von Startsubstanz und Spuren von CBD.

2,07 ml Triethylamin (C₂H₅)₃N (1,5 Äquivalent) werden zugegeben und die Suspension 40 Stunden lang gerührt. DC zeigt CBD als Hauptprodukt mit Spuren des Monobrom-Derivats.

Das Gemisch wird bis Raumtemperatur gekühlt. 4,2 ml Essigsäure (ca. 7,2 Äquivalente) werden tropfenweise zugegeben (bis pH-Wert im Bereich von 6 -7 liegt). Das Gemisch wird mit 40 ml Methanol verdünnt und über eine Filterfritte abgesaugt. Der Filterkuchen wird mit 20 ml Methanol gewaschen. Das Filtrat wird dreimal mit 150 ml n-Heptan extrahiert. Die vereinten n-Heptan-Fraktionen werden mit 100 ml VE-Wasser gewaschen und mit Natriumsulfat getrocknet. Die entstehende Suspension wird über eine Filterfritte abgesaugt, und Feststoff wird mit 30 ml n-Heptan gewaschen. Das leicht-gelbe Filtrat am Rotationsverdampfer wird bei einer Badtemperatur von 40°C bis Enddruck von 20 mbar eingeengt. Der erhaltene Rest wird aus 2 ml n-Heptan umkristallisiert. Masse des Endproduktes = 649,3 mg (Gesamtausbeute nach 2 Stufen = 20%).

Als weitere Basen zur Debromierung eignen sich Diisopropyl-Ethlyamin (C₈H₁₉)N, Triethylamin (C₂H₅)₃N und/oder Diazabicycloundecen (DBU, C₉H₁₆N₂).

### Analytische Daten:

¹H-NMR (CDCl₃): δ = 0.88 (t, 3H, J = 7,0 Hz), 1.29 (m., 4H), 1.55 (m, 2H), 1.66 (s, 3H), 1.79 (s, 3H), 1.82 (m, 2H), 2.08 (m, 1H), 2.23 (m, 1H), 2.42 (m, 3H), 3.85 (m, 1H), 4.56 (s, 1H), 4.66 (br. s., 2H), 5.57 (d, 1H, *J* = 1,2 Hz), 5.98 (br. s., 1H), 6.16 (br. s., 1H), 6.28 (br. s., 1H).

HPLC: 99, 8% Reinheit. [α]_{D} = -130,6° (c = 0,49 in Ethanol).

Das Auftreten von Stereoisomeren kann mittels NMR und HPLC nicht nachgewiesen werden.

### BEZUGSZEICHENLISTE

- 1: Olivetol (5-Pentylresorcin)
- 2: Di-OTBS-Olivetol (Di-O*-tert-*Butyldimethylsilyl-5-Pentylresorcin)
- 3: TBSCI (tert-Butyldimethylsilylchlorid)
- 4: Di-OTBS-dibromolivetol (4,6-Dibrom-di-O-tert-Butyldimethylsilyl-5-Pentylresorcin)
- 5: N-Bromosuccinimid
- 6: Dibromolivetol (4,6-Dibrom-5-Pentylresorcin)

- 7: pTSA (para-Toluolsulfonsäure-Monohydrat
- 8: trans-Menthadienol (4R-Isopropenyl-1-methylcyclohex-2-enol)
- 9: Dibromcannabidiol
- 10: Cannabidiol (2-[(1R,6R)-3-Methyl-6-prop-1-en-2-yl-1-cyclohex-2-enyl]-5-pentylbenzo-1,3-diol)

## Patentansprüche

1. Verfahren zur Herstellung von Cannabidiol (10) (2-[(1R,6R)-3-Methyl-6-prop-1-en-2-yl-1-cyclohex-2-enyl]-5-pentylbenzo-1,3-diol) oder seinen pentylsubstituierten Derivaten **gekennzeichnet durch**
- Silylieren von am Pentlyrest substituiertem oder unsubstituiertem Olivetol (5-Pentylresorcin) (1) mit mit TBSCI *(tert-*Butyldimethylsilylchlorid) (3), gemäß folgendem Reaktionsschema 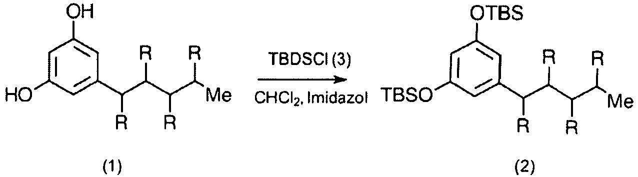 wobei die Pentylsubstituenten substituiert sind mit Substituenten ausgewählt aus der Gruppe bestehend aus:
Wasserstoff (H), Deuterium (D) oder Tritium (T), Alkoxy wie Methoxy (-OCH₃), Ethoxy (*-*OC₂H₅), Nitro (-NO₂), Fluor (-F), Chlor (-CI), Brom (-Br), Alkyl von Methyl, Ethyl, Propyl bis Butyl,
wodurch ein entsprechend unsubstituiertes (R = H) oder Di-O-*tert-*Butyldimethylsilyl-5-Pentylresorcin (2) erhalten
- Bromieren des im ersten Schritt erhaltenen und gegebenenfalls substituierten Di-O-*tert*-Butyldimethylsilyl-5-Pentylresorcins (2) zu Di-OTBSdibromolivetol (4,6-Dibrom-di-O-*tert-*Butyldimethylsilyl-5-Pentylresorcin),
- Desilylieren des im vorhergenden Schritts erhaltenen und gegebenenfalls substituierten Di-OTBS-dibromolivetol (4,6-Dibrom-di-O-tert-Butyldimethylsilyl-5-Pentylresorcin) zu Dibromolivetol (4,6-Dibrom-5-Pentylresorcin) oder dem entsprechend substituierten Derivats,
- Umsetzen des im vorhergehenden Schritt erhaltenen Dibromolivetol (4,6-Dibrom-5-Pentylresorcin) oder dem entsprechend substituierten Derivats mit *trans*-Menthadienol (4R-Isopropenyl-1-methylcyclohex-2-enol) (8) zu Dibromcannabidiol (9) oder seinem entsprechend substituierten Derivats,
- Debromieren des im vorhergehenden Schritt erhaltenem Dibromcannabidiol (9) oder seinem entsprechend substituierten Derivat zu Cannabidiol oder seinem entsprechenden Derivat.

2. Verfahren nach Anspruch 1
**gekennzeichnet durch**
Silylieren im ersten Schritt in Gegenwart von Imidazol in Dichlormethan (CH₂Cl₂).

3. Verfahren nach einem der Ansprüche 1 oder 2,
**gekennzeichnet durch**
Bromieren mit N-Bromosuccinimid in Dichlormethan (CH₂Cl₂).

4. Verfahren nach einem der Ansprüche 1 bis 3,
**gekennzeichnet durch**
Desilylieren durch saure Hydrolyse in Tetrahydrofuran.

5. Verfahren nach einem der Ansprüche 1 bis 4,
**gekennzeichnet durch**
Umsetzen zu Dibromcannabidiol in Dichlormethan (CH₂Cl₂) unter Gegenwart von para-Toluolsulfonsäure.

6. Verfahren nach einem der Ansprüche 1 bis 5,
**gekennzeichnet durch**
Debromieren des Dibromoicannabidiols in Methanol in Gegenwart von mindestens einer Base ausgesucht aus der Gruppe bestehen aus Ammoniak (NH₃), Diisopropyl-Ethlyamin (C₈H₁₉)N, Triethylamin (C₂H₅)₃N und/oder Diazabicycloundecen (DBU, C₉H₁₆N₂).

7. Silyliertes Olivetol mit folgender Struktur 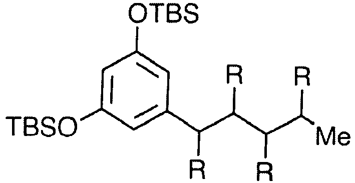 mit
OTBS O-*tert*-Butyldimethylsilyl, und
je ein Rest R ausgesucht ist aus der Gruppe bestehend aus Wasserstoff (H), Deuterium (D) oder Tritium (T), Alkoxy, wie Methoxy (-OCH3), Ethoxy (-OC2H5), Nitro (-NO₂), Fluor (-F), Chlor (-CI), Brom (-Br), Alkyl von Methyl, Ethyl, Propyl bis Butyl.

8. Silyliertes und bromiertes Olivetol mit folgender Struktur 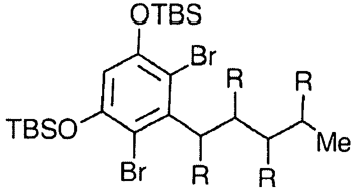 mit
OTBS O-*tert*-Butyldimethylsilyl, und
je ein Rest R ausgesucht ist aus der Gruppe bestehend aus Wasserstoff (H), Deuterium (D) oder Tritium (T), Alkoxy, wie Methoxy (-OCH₃), Ethoxy (-OC₂H₅), Nitro (-NO₂), Fluor (-F), Chlor (-CI), Brom (-Br), Alkyl von Methyl, Ethyl, Propyl bis Butyl.

## Claims

1. A method for the manufacture of cannabidiol (10) (2-[(1R,6R)-3-methyl-6-prop-1-en-2-yl-1-cyclohex-2-enyl]-5-pentylbenzo-1,3-diol)or pentyl-substituted derivatives thereof,
**characterized by**
- silylation of substituted or unsubstituted olivetol (5-pentylresorcinol) (1) in the pentyl residue with TBSCl (*tert*-butyldimethylsilyl chloride (3), according to the following reaction diagram 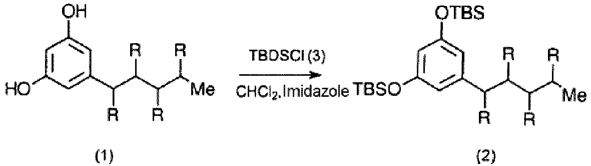 wherein the pentyl substituents are substituted with substituents chosen from the group made up of:
hydrogen (H), deuterium (D) or tritium (T), alkoxy, such as methoxy (-OCH₃), ethoxy (-OC₂H₅), nitro (-NO₂), fluorine (-F), chlorine (-Cl), bromine (-Br), alkyl of methyl, ethyl, propyl to butyl,
as a result of which a correspondingly unsubstituted (R = H) or Di-O-*tert*-butyldimethylsilyl-5-pentylresorcinol (2) is obtained
- bromination of the Di-O-*tert*-butyldimethylsilyl-5-pentylresorcinol (2) obtained in the first step and possibly substituted to Di-OTBS-dibromolivetol (4,6-dibromo-di-O-*tert*-butyldimethylsilyl-5-pentylersorcinol),
- desilylation of the Di-OTBS-dibromolivetol (4,6-dibromo-di-O-*tert-*butyldimethylsilyl-5-pentylresorcinol) obtained in the preceding step and possibly substituted to dibromolivetol (4,6-dibromo-5-pentylresorcinol) or the correspondingly substituted derivative,
- conversion of the dibromolivetol obtained in the preceding step (4,6-dibromo-5-pentylresorcinol) or the correspondingly substituted derivative with *trans-*menthadienol (4R-isopropenyl-1-methylcyclohex-2-enol) (8) into dibromo-cannabidiol (9) or its correspondingly substituted derivative,
- debromation of the dibromo-cannabidiol (9) obtained in the preceding step or its correspondingly substituted derivative into cannabidiol or its corresponding derivative.

2. The method according to claim 1,
**characterized by**
silylation in the first step in the presence of imidazole in dichloromethane (CH₂Cl₂).

3. The method according to one of claims 1 or 2,
**characterized by**
bromation with N-bromo-succinimide in dichloromethane (CH₂Cl₂).

4. The method according to one of claims 1 to 3,
**characterized by**
desilylation through acid hydrolysis in tetrahydrofuran.

5. The method according to one of claims 1 to 4,
**characterized by**
conversion to dibromo-cannabidiol in dichloromethane (CH₂Cl₂) in the presence of para-toluenesulfonic acid.

6. The method according to one of claims 1 to 5,
**characterized by**
debromation of the dibromo-cannabidiol in methanol in the presence of at least one base selected from the group made up of ammonium (NH₃), diisopropylethylamine (C₈H₁₉)N, triethylamine (C₂H₅)₃N and/or diazabicycloundecene (DBU, C₉H₁₆N₂).

7. Silylated olivetol having the following structure 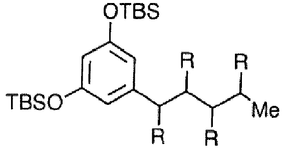 where
OTBS O-*tert*-butyldimethylsilyl, and
a residue R in each case selected from the group comprising hydrogen (H), deuterium (D) or tritium (T), alkoxy, such as methoxy (-OCH₃), ethoxy (-OC₂H₅), nitro (-NO₂), fluorine (-F), chlorine (-Cl), bromine (-Br), alkyl of methyl, ethyl, propyl to butyl.

8. Silylated and bromated olivetol having the following structure 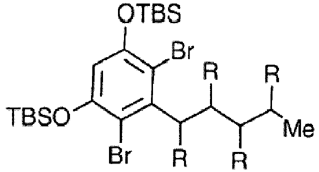 where
OTBS O-*tert*-butyldimethylsilyl, and
a residue R in each case selected from the group comprising hydrogen (H), deuterium (D) or tritium (T), alkoxy, such as methoxy (-OCH₃), ethoxy (-OC₂H₅), nitro (-NO₂), fluorine (-F), chlorine (-Cl), bromine (-Br), alkyl of methyl, ethyl, propyl to butyl.

## Revendications

1. Procédé de préparation de cannabidiol (10) (2-[(1R,6R)-3-Methyl-6-prop-1-en-2-yl-1-cyclohex-2-enyl]-5-pentylbenzo-1,3-diol) ou de ses dérivés substitués aux groupes pentyle,
**caractérisé en ce que**
- l'olivétol (5-pentylresorcine) (1), dont le radical pentyle est substitué ou non-substitué, subit une silylation avec du TBSCI (chlorure de *tert-*butyldiméthylsilyle) (3) selon le schéma de réaction suivant 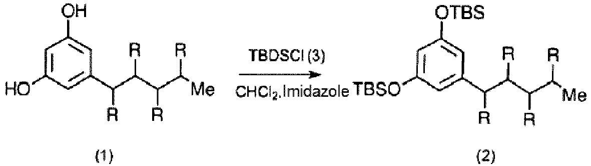 les substituants du groupe pentyle étant substitués avec des substituants choisis dans le groupe constitué de :
hydrogène (H), deutérium (D) ou tritium (T), alcoxy tel que méthoxy (-OCH₃), éthoxy (-OC₂H₅), nitro (-NO₂), fluoro (-F), chloro (-Cl), bromo (-Br), alkyle allant de méthyle, éthyle, propyle jusqu'à butyle,
pour ainsi obtenir une di-O-*tert*-butyldiméthylsilyl-5-pentylresorcine (2) correspondant qui peut être non-substituée (R = H)
- la di-O-*tert*-butyldiméthylsilyl-5-pentylresorcine (2) obtenue à la première étape, et le cas échéant substituée, est soumise à une bromation pour devenir du di-OTBS-dibromo-olivétol (4,6-dibromo-di-O-*tert-*butyldiméthylsilyl-5-pentylresorcine),
- le di-OTBS-dibromo-olivétol (4,6-dibromo-di-O-*tert*-butyldiméthylsilyl-5-pentylresorcine) obtenu à l'étape précédente, et le cas échéant substitué, est soumis à une désilylation pour devenir du dibromo-olivétol (4,6-dibromo-5-pentylrésorcine) ou le dérivé substitué correspondant,
- le dibromo-olivétol (4,6-dibromo-5-pentylrésorcine obtenu à l'étape précédente, ou le dérivé substitué correspondant, est soumis à une réaction avec du trans-menthadiénol (4R-isopropényl-1-méthylcyclohex-2-énol) (8) pour devenir du dibromocannabidiol (9) ou son dérivé substitué correspondant,
- le dibromocannabidiol (9) obtenu à l'étape précédente, ou son dérivé substitué correspondant, est soumis à une débromation pour devenir du cannabidiol ou son dérivé substitué correspondant.

2. Procédé selon la revendication 1,
**caractérisé en ce que**
à la première étape, la silylation est réalisée en présence d'imidazole dans du dichlorométhane (CH₂Cl₂).

3. Procédé selon l'une des revendications 1 ou 2,
**caractérisé en ce que**
la bromation est réalisée avec du N-bromosuccinimide dans du dichlorométhane (CH₂Cl₂).

4. Procédé selon l'une des revendications 1 à 3,
**caractérisé en ce que**
la désilylation est réalisée par hydrolyse acide dans du tétrahydrofurane.

5. Procédé selon l'une des revendications 1 à 4,
**caractérisé en ce que**
la réaction donnant du dibromocannabidiol est réalisée dans du dichlorométhane (CH₂Cl₂) en présence de l'acide para-toluènesulfonique.

6. Procédé selon l'une des revendications 1 à 5,
**caractérisé en ce que**
la débromation du dibromocannabidiol est réalisée dans du méthanol en présence d'au moins une base choisie dans le groupe constitué d'ammoniac (NH₃), de diisopropyl-éthylamine (C₈H₁₉)N, de triéthylamine (C₂H₅)₃N et/ou de diazabicyclo-undécène (DBU, C₉H₁₆N₂).

7. Olivétol silylé ayant la structure suivante 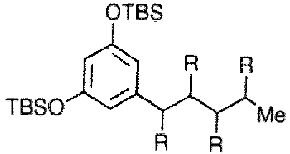 avec
OTBS O-*tert*-butyldiméthylsilyle, et
à chaque occurrence, l'un des radicaux R étant choisi dans le groupe constitué d'hydrogène (H), deutérium (D) ou de tritium (T), d'alcoxy tel que méthoxy (-OCH₃), éthoxy (-OC₂H₅), de nitro (-NO₂), de fluoro (-F), chloro (-Cl), bromo (-Br), d'alkyle allant de méthyle, éthyle, propyle jusqu'à butyle.

8. Olivétol silylé et bromé ayant la structure suivante 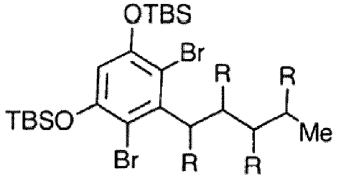 avec
OTBS O-*tert*-butyldiméthylsilyl, et
à chaque occurrence, l'un des radicaux R étant choisi dans le groupe constitué d'hydrogène (H), deutérium (D) ou de tritium (T), d'alcoxy tel que méthoxy (-OCH₃), éthoxy (-OC₂H₅), de nitro (-NO₂), de fluoro (-F), chloro (-Cl), bromo (-Br), d'alkyle allant de méthyle, éthyle, propyle jusqu'à butyle.
